Europäisches Patentamt

European Patent Office

Office européen des brevets

(11) Publication number: **0 141 244**
**B1**

(12) **EUROPEAN PATENT SPECIFICATION**

(45) Date of publication of patent specification: **07.12.88**

(21) Application number: **84111426.7**

(22) Date of filing: **25.09.84**

(51) Int. Cl.⁴: **C 12 Q 1/28,** C 12 Q 1/54, **G 01 N 33/72**

(54) **Testing agent for detecting a subtance in a body fluid.**

(30) Priority: **18.10.83 JP 194937/83**

(43) Date of publication of application:
**15.05.85 Bulletin 85/20**

(45) Publication of the grant of the patent:
**07.12.88 Bulletin 88/49**

(84) Designated Contracting States:
**DE FR IT**

(56) References cited:
**EP-A-0 037 056**
**EP-A-0 041 188**
**EP-A-0 103 958**
**US-A-3 411 887**
**CLINICAL CHEMISTRY, vol. 28, no. 4, April 1982, pages 578-588, Washington, US; R.H. WHITE-STEVENS: "Interference by ascorbic acid in test systems involving peroxidase. I. Reversible indicators and the effects of copper, iron and mercury"**
**The file contains technical information submitted after the application was filed and not included in this specification**

(73) Proprietor: **TERUMO KABUSHIKI KAISHA trading as TERUMO CORPORATION**
**44-1, 2-chome, Hatagaya Shibuya-Ku**
**Tokyo 151 (JP)**

(72) Inventor: **Kaminagayoshi, Satoshi**
**Corpo Maruzen 202 Suge 2474 Tama-ku**
**Kawasaki-shi Kanagawa-Ken (JP)**

(74) Representative: **Henkel, Feiler, Hänzel & Partner**
**Möhlstrasse 37**
**D-8000 München 80 (DE)**

Courier Press, Leamington Spa, England.

# EP 0 141 244 B1

**Description**

Background of the invention
Field of the invention:

This invention relates to a testing agent for the detection of glucose in urine and blood and for the detection of blood in urine, faeces and a body fluid.

Description of prior art:

Recently, quick diagnostic agents are gaining remarkably in significance. Those quick diagnostic agents which are based on absorbent carriers are important. Such an agent is prepared by impregnating an absorbent carrier, which is a strip of paper in most cases, with a chemical reagent necessary for the reaction utilized for the detection, and the agent is immersed in a given body fluid. The agent assumes a color reaction in the presence of the substance being detected. Quick diagnostic agents which have become extremely important for medical examinations are those to be used for the detection of glucose in urine and blood and for the detection of blood in urine, faeces and a body fluid.

The test under discussion is based on the following principle. In the detection of dextrose in body fluid, for example, glucose is oxidized by glucose oxidase (GOD) into gluconic acid and, in the meantime, the gaseous oxygen is reduced into hydrogen peroxide. This hydrogen peroxide oxidizes an oxidative indicator into a corresponding coloring matter through the agency of a peroxidase or a peroxidase-acting substance. The density of the color of this coloring matter serves as the scale of the amount of glucose present. In the detection of blood, the peroxidase-like activity of hemoglobin causes release of oxygen from the peroxide contained in the test paper and consequently oxidizes the oxidative indicator.

Generally, oxidative indicators for use in such quick diagnostic agents as mentioned above are known to the art and belong to a specific class of compounds. Particularly useful are benzidine type heterocyclic azines and phenols, particularly the component of guaiacum resin (Japanese Patent Laid-Open SHO 53 (1978)-126,996).

Recent processed foodstuffs and refreshing beverages contain ascorbic acid as vitamin C in large amounts. Further, vitamin preparations are consumed in large amounts for the preservation of health. Consequently, users of such preparations carry such vitamins in large proportions in their blood. Excess vitamins are destined to be excreted in urine. When the test is performed on urine or blood by the use of the diagnostic agent, since ascorbic acid possesses high reducing activity, the oxygen formed by the agency of peroxidase or a peroxidase-acting substance is consumed in the oxidation-reduction reaction with ascorbic acid and the reaction of the oxidative indicator is inhibited and prevented from assuming a color reaction sufficiently.

EP—A—0 103 958 discloses an immuno assay wherein a peroxidase catalyzes the formation of a dye and ascorbate interference (with the assay) is reduced or eliminated by the presence of a periodate. However, due to the presence of interfering components which would render the respective detection inaccurate, the said immuno assay is not suitable for detecting glucose or occult blood in a sample of a body fluid.

From EP—A—0 037 056 there is known a testing system for the detection of redox reactions, e.g. of glucose in a sample of a body fluid, comprising glucose oxidase and peroxidase, a coloring agent which serves to detect the oxidation product as formed and an iodate ($IO_3^-$) to reduce or eliminate an interference by ascorbic acid.

Object of the invention

An object of this invention, therefore, is to provide a novel testing agent excellent in stability and suitable for the detection of glucose in urine and blood and the detection of blood in urine, faeces and a body fluid.

Summary of the invention

The objects described above are accomplished by a testing agent for detecting of glucose or occult blood in a sample of a body fluid which may also contain a reducing substance, which comprises

(A) (i) a hydroperoxide or hydrogen peroxide (for the occult blood test) or
(ii) a peroxidase and a glucose oxidase (for the glucose test)
(B) an effective amount sufficient to oxidize the reducing substance in said body fluid of $NaIO_4$ and
(C) a coloring agent which serves to detect the oxygen produced by the peroxidase or peroxidase-like reaction.

Further, this invention is directed to a testing agent wherein said effective amount of the $NaIO_4$ is in the range of 0.3 to 1.000 m · mol/liter. This invention is directed to a testing agent wherein said testing agent is a solution of an agent containing said coloring agent and $NaIO_4$; said $NaIO_4$ is an amount of from 0.3 to 1.000 m · mol/liter; and said coloring agent is at least one member selected from the group consisting of benzidine type compounds, heterocyclic azines, phenols and guaiacum resin component. This invention is directed to a testing agent wherein said $NaIO_4$ is an amount of from 0.5 to 40 m · mol/liter and wherein said coloring agent is o-tolidine or phenol. This invention is directed to a testing agent wherein the component (A) is glucose oxidase. This invention is directed to a testing agent wherein said component (A) is a

2

hydroperoxide selected from the group consisting of 2,5 - dimethylcyclohexane - 2,5 - dihydroperoxide, cumene hydroperoxide, 2,5 - dimethyl - cyclohexanone - 2,5 - dihydroperoxide, diisopropylbenzene hydroperoxide, t - butyl - hydroperoxide, p-menthane hydroperoxide and 4 - methylphenylisopropyl hydroperoxide. This invention is directed to a testing agent wherein said hydroperoxide is 2,5 - dimethylcyclohexanone - 2,5 - dihydroperoxide or cumene hydroperoxide. This invention is directed to a testing agent in the form of a solution which comprises the components (A), (B) and (C).

Preferred embodiment of the invention

The testing agent provided in this invention is intended for the detection of glucose in urine and blood and for the detection of blood in urine, faeces and a body fluid. In the testing agent, the $NaIO_4$ is incorporated therein for the purpose of preventing the testing agent from being affected by the aforementioned reducing substance.

The $NaIO_4$ is used in an amount such that, during the storage of the testing agent, it does not oxidize the oxidative indicator and, during the use of the testing agent for the aforementioned detection, it effectively prevents the reaction of the oxidative indicator from the inhibition caused by such reducing substance as ascorbic acid present in the fluid under test. To be specific, the $NaIO_4$ is used generally in an amount of 0.3 to 1.000 m · mol/liter, preferably in an amount of 0.5 to 40 m · mol/liter, though variable with the amount of the reducing substance contained in the fluid under test.

The coloring agent which is oxidized and enabled to color serves to detect the oxygen produced by the peroxidase or peroxidase-like reaction and, in that capacity, participates in the reaction producing oxidized condensates (phenol, with 4-aminoantipyrine, etc.), the reaction causing methanol to be oxidized into formaldehyde and then combined with acetylacetone to produce a condensate, and the reaction causing an iodide such as potassium iodide to be oxidized to produce iodine.

As coloring agents, benzidine type compounds, heterocyclic azines, phenols and guaiacum resin components are known to the art. Typical examples include o-tolidine, m-tolidine, bendizine, 3,3',5,5'-tetramethyl benzidine, o-methyl benzidine, 2,7-diaminofluorene, mixtures containing these compounds in various proportions, various substituted phenylene diamines such as 4,4'-diaminodiphenyl, o-phenylene diamine, m-phenylene diamine, p-phenylene diamine, 2,3-tolylene diamine, 2,4-tolylene diamine, 2,5-tolylene diamine and 2,6-tolylene diamine, pyrogallic acid, gallic acid, phenols such as fluoroglucinol, hydroquinone, and leucoindophenol, guaiacol, pyridine derivatives, substituted azines and leuco-malachite green. The coloring agent is used generally in an amount of 2 to 250 m · mol/liters, preferably in an amount of 9 to 50 m.mol/liter.

Examples of the hydroperoxide to be used in this invention include 2,5 - dimethylcyclohexane - 2,5 - dihydroperoxide, cumene hydroperoxide, 2,5 - dimethylcyclohexanone - 2,5 - dihydroperoxide, diisopropylbenzene hydroperoxide, t-butyl hydroperoxide, p-menthane hydroperoxide and 4 - methyl-phenylisopropylhydroperoxide. Among other hydroperoxides enumerated above, 2,5 - dimethylcyclohexanone - 2,5 - dihydroperoxide and cumene hydroperoxide are desirable hydroperoxides having relatively low decomposition ratios.

The testing agent of the present invention is used in the form of solution or in the form of test piece having a substrate such as paper impregnated with the solution. The testing agent may optionally incorporate therein a buffer or a lubricant. The buffer serves to retain the pH value of the fluid in which the test piece is immersed in the range of 4 to 8, preferably 5 to 7. Examples of the buffer are citric acid-sodium citrate, tartaric acid-sodium tartrate, malic acid-borax, potassium hydrogen phthalate-dipotassium phthalate and sodium hydrogen succinate-disodium succinate.

The wetting agent serves to enable the body fluid in which the test piece is immersed to uniformly wet the test piece. Examples of the wetting agent are surface active agents including alkyl sulfonic acid salts such as sodium lauryl sulfate, sodium dodecyl sulfate and sodium tetradecyl sulfate, alkylbenzene sulfonates such as sodium dodecylbenzene sulfonate and dialkylsulfosuccinates such as sodium dioctyl-sulfosuccinate and sodium diheptylsulfosuccinate.

Further, the aforementioned testing agent may incorporate therein a chemical agent capable of enhancing the peroxidase activity in hemoglobin. Typical enhancers usable for this purpose include quinoline and derivatives thereof, such as quinine, cinchonine, o - methoxyquinoline, quinalidine, 8 - amino - 6 - methoxy quinoline, 2 - quinolinol, isoquinoline, benzo(f)quinoline and 3 - amino - quinoline. In the presence of such an enhancer, the oxidation reaction is generally accelerated and the oxidized chromogen has its color-generating strength enhanced. Consequently, the test piece acquires increased sensitivity.

When the testing agent is to be used in the form of test piece which has a substrate impregnated with the testing agent, it may incorporate therein an adhesive agent for the purpose of preventing exudation of the agent from the test piece. Typical adhesive agents are polymers such as polyvinyl alcohol, polyvinyl pyrrolidone, polyethylene glycol, polyacrylates, polyacrylamide, poly(hydroxyethyl methacrylate), poly(hydroxyethyl acrylate), carboxymethyl cellulose and gelatin and gum arabic.

The aforementioned testing agent is used in the form a solution. Examples of the solvent to be used in the preparation of such a solution are benzene, toluene, xylene, methanol, ethanol, isopropanol, acetone, methylethyl ketone, chloroform, carbon tetrachloride and water. Otherwise, the testing agent is used in the form of a test piece which is obtained by impregnating a substrate with the aforementioned solution and drying the wet substrate. The test piece is manufactured by a conventional method.

As the substrate for the test piece, filter paper, glass fibers and non-woven fabric made of plastic material are available. The substrate so selected fits the preparation of the test piece when it fulfils the requirement that it should neither react upon nor dissolve in the impregnating solution and should possess an absorbing property.

The testing agent of the present invention is used for the detection of glucose or occult blood.

Now, this invention will be described more specifically below with reference to working examples.

Example 1 and Control 1
Glucose test paper

| Solution A | |
|---|---|
| Citrate buffer | 100 ml |
| Glucose oxidase | 300 mg |
| Peroxidase | 50 mg |
| $NaIO_4$ | 100 mg |
| Sodium alginate (1% solution) | 500 mg |

| Solution B | |
|---|---|
| Ortho-tolidine | 2.5 g/100 ml acetone |

A glucose test paper was obtained by impregnating a filter paper (made by Toyo Roshi Co.) with Solution A, drying the wet filter paper, again impregnating the filter paper with Solution B, and drying the wet filter paper.

The test paper thus prepared were immersed in two types of urine specimens each containing 50 mg of ascorbic acid; one type containing no glucose and the other containing 150 mg of glucose per dl. The results were as shown in Table 1.

TABLE 1

| Example | Oxidizing agent | Urine (containing no glucose) | Urine (containing 150 mg of glucose per dl) |
|---|---|---|---|
| 1 | $NaIO_4$ | − | + (150 mg/dl) |
| Control 1 | None | − | − |

Example 2 and Control 2
Glucose test solution

| Solution A | |
|---|---|
| Glucose oxidase | 3.25 U/ml |
| Peroxidase | 0.3 U/ml |
| $NaIO_4$ | 1 mg/ml |

| Solution B | |
|---|---|
| 4-Aminoantipyrine | 5 mg/dl |
| Phenol | 50 mg/dl |

A glucose test solution was prepared by dissolving Solution A in 90 ml of 0.1 M phosphate buffer solution (pH 6.9) and then dissolved Solution B in the resultant solution.

A 3-ml portion of the test solution thus prepared and 20 μl of an aqueous glucose solution containing 5 mg of glucose and 50 mg of ascorbic acid each per dl were mixed and heated at 37°C for 20 minutes. The resultant reaction solution was tested for absorbance at 505 nm. The results were as shown in Table 2.

TABLE 2

| Example | Oxidizing agent | Glucose (none) | Glucose (75 mg/dl) |
|---|---|---|---|
| 2 | $NaIO_4$ | 0 | 77 mg/dl |
| Control 2 | None | 0 | 21 mg/dl |

4

Example 3

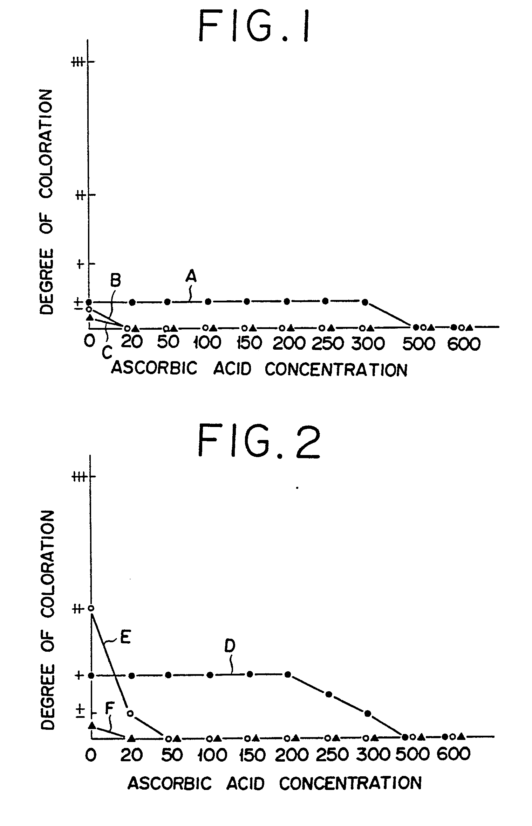

By using the glucose testing paper obtained in Example 1, urine specimens invariably containing 50 mg of glucose per dl and containing varying amounts of ascorbic acid were tested to find the relation between the ascorbic acid concentration and the degree of coloration. The results were as shown in Fig. 1. In the graph, the curve A represent the data obtained by using the test paper of Example 1, the curve B the data obtained by using a commercially available glucose test paper I, and the curve the data obtained by using another commercially available glucose test paper II.

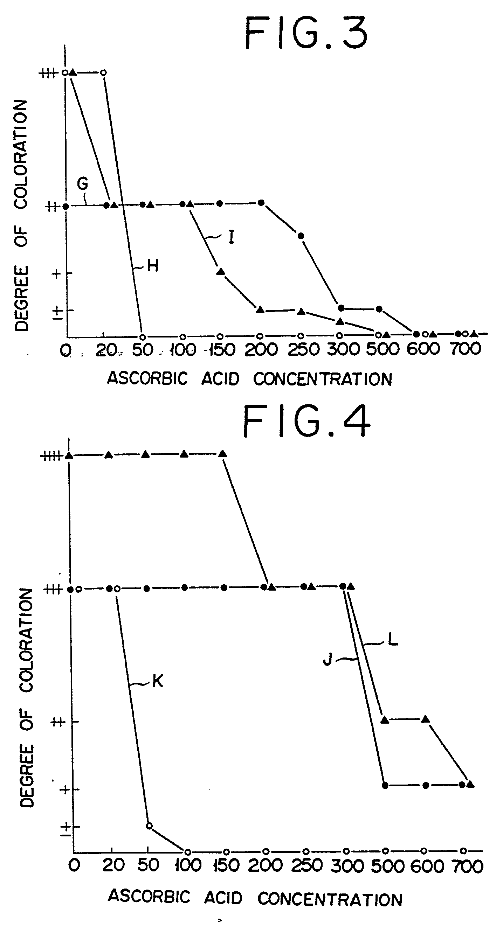

The same test was performed on urine specimens containing 150 mg, 500 mg and 2000 mg respectively of glucose per dl and varying amounts of ascorbic acid, to determine the relation between the ascorbic acid concentration and the degree of coloration. The results were as shown in Figs. 2—4 respectively. In the graphs, the curves D, G and J represent the data obtained by using the test paper of Example 1, the curves E, H and K the data obtained by using a commercially available glucose test paper I, and the curves F, I and L the data obtained by using another commercially available glucose test paper II.

In these graphs, the mark $\pm$ indicated along the vertical axis represents a glucose concentration of 50 mg/dl, the mark $+$ a glucose concentration of 150 mg/dl, the mark $++$ a glucose concentration of 500 mg/dl, and the mark $+++$ a glucose concentration of 2000 mg/dl respectively. The commercially available glucose test paper I contained 25 mg of citric acid, 146 mg of sodium citrate, 48 mg of glucose oxidase, 0.6 mg of peroxidase, 6 mg of ortho-tolidine and 10.25 mg of eosin per 100 sheets. The other commercially available glucose test paper II contained 300 µl of glucose oxidizing enzyme, 0.9 mg of peroxidase and 9 mg of potassium iodide in addition to the components of test paper per 100 sheets.

Example 4
Occult blood test paper

| Solution A | |
|---|---|
| 2,5-Dimethylhexane-2,5-dihydroperoxide | 1.2 g |
| Ethanol | 100 ml |

| Solution B | |
|---|---|
| Sodium citrate | 9 g |
| Citric acid | 1 g |
| Saponin | 100 mg |
| $NaIO_4$ | 200 mg |
| Polyethylene glycol (m.w. 4000) | 10 g |
| Water | 100 ml |

| Solution C | |
|---|---|
| Ortho-tolidine | 1.2 g |
| Benzene | 100 ml |
| Aminoquinoline (sensitizer) | 0.5 g |

An occult blood test paper was produced by impregnating filter paper with solution A, drying the wet filter paper, again impregnating the paper with Solution B, drying the paper, and finally impregnating the paper with Solution C, and drying.

The test paper was immersed in urine specimens containing hemoglobin in varying concentrations by way of test for occult blood. Depending on the hemoglobin concentrations, the test papers assumed blue color in varying intensities. When the test was repeated in the same urine specimens now additionally containing ascorbic acid (in concentrations up to 200 mg/dl), ascorbic acid manifested absolutely no effect.

As described above, the testing agent of the present invention which is useful for the detection of glucose or occult blood in a sample of a body fluid which may also contain a reducible substance, which comprises (A) one member selected from the group consisting of (i) a hydroperoxide or hydrogen peroxide and (ii) a peroxidase or a glucose oxidase, (B) an effective amount sufficient to oxidize the reducible substance in said body fluid of $NaIO_4$ and (C) a coloring agent which serves to detect the oxygen produced by the peroxidase or peroxidase-like reaction.

Even when the fluid under test such as urine or blood contains a reducing substance such as ascorbic acid or gentisic acid, therefore, the peroxidase or other peroxidase-like reaction is not impeded because the reducing substance is oxidized by the $NaIO_4$ agent. Even when the $NaIO_4$ is contained in advance in the testing agent, the peroxidase or other peroxidase-like reaction is not promoted. The effect is particularly manifest when the $NaIO_4$ is used in an amount of 0.3 to 1,000 m · mol/liter.

**Claims**

1. A testing agent for detecting of glucose or occult blood in a sample of a body fluid which may also contain a reducing substance, which comprises

5

(A) (i) a hydroperoxide or hydrogen peroxide (for the occult blood test) or

(ii) a peroxidase and a glucose oxidase (for the glucose test)

(B) an effective amount sufficient to oxidize the reducing substance in said body fluid of $NaIO_4$ and

(C) a coloring agent which serves to detect the oxygen produced by the peroxidase or peroxidase-like reaction.

2. A testing agent according to Claim 1, wherein said effective amount of the $NaIO_4$ is in the range of 0.3 to 1,000 m · mol/liter.

3. The testing agent according to Claim 1, wherein said testing agent is a solution of an agent containing said coloring agent and $NaIO_4$; said $NaIO_4$ is in an amount of from 0.3 to 1,000 m · mol/liter; and said coloring agent is at least one member selected from the group consisting of benzidine type compounds, heterocyclic azines, phenols and guaiacum resin components.

4. The testing agent according to Claim 3, wherein said $NaIO_4$ is in an amount of from 0.5 to 40 m · mol/liter and wherein said coloring agent is o-tolidine or phenol.

5. The testing agent according to any one of Claims 1 to 4, wherein said component (A) is a hydroperoxide selected from the group consisting of 2,5 - dimethylcyclohexane - 2,5 - dihydroperoxide, cumene hydroperoxide, 2,5 - dimethylcyclohexanone - 2,5 - dihydroperoxide, diisopropylbenzene hydroperoxide, t - butyl hydroperoxide, p-menthane hydroperoxide and 4 - methylphenylisopropyl hydroperoxide.

6. The testing agent according to Claim 5, wherein said hydroperoxide is 2,5 - dimethylcyclohexanone - 2,5 - dihydroperoxide or cumene hydroperoxide.

7. The testing agent according to any one of Claims 1 to 6 in the form of a solution which comprises the components (A), (B) and (C).

8. A testing agent which is in the form of a test paper produced by impregnating a substrate with the solution of any one of Claims 1 to 7.

**Patentansprüche**

1. Testmittel zum Nachweis von Glucose oder Okkultblut in einer gegebenenfalls eine reduzierende Substanz enthaltenden Probe einer Körperflüssigkeit, enthaltend

(A) (1) ein Hydroperoxid oder Wasserstoffperoxid (für den Okkultbluttest) oder

(2) eine Peroxidase und eine Glucoseoxidase (für den Glucosetest),

(B) eine zur Oxidation der reduzierenden Substanz in der Körperflüssigkeit ausreichende, wirksame Menge $NaIO_4$ und

(C) ein zum Nachweis des durch die Peroxidase oder peroxidaseartige Reaktion gebildeten Sauerstoffs dienendes Färbemittel.

2. Testmittel nach Anspruch 1, dadurch gekennzeichnet, daß die wirksame Menge $NaIO_4$ im Bereich von 0,3 bis 1000 mMol/l liegt.

3. Testmittel nach Anspruch 1, dadurch gekennzeichnet, daß das Testmittel eine Lösung eines Mittels mit dem Färbemittel und $NaIO_4$ ist, wobei die $NaIO_4$-Menge 0,3 bis 1000 mMol/l beträgt und das Färbemittel aus mindestens einem Bestandteil aus der Gruppe Benzidinverbindungen, heterocyclische Azine, Phenole und Guajakharzkomponenten besteht.

4. Testmittel nach Anspruch 3, dadurch gekennzeichnet, daß die $NaIO_4$-Menge 0,5 bis 40 mMol/l beträgt und das Färbemittel aus o-Tolidin oder Phenol besteht.

5. Testmittel nach einem der Ansprüche 1 bis 4, dadurch gekennzeichnet, daß die Komponente (A) aus einem Hydroperoxid aus der Gruppe: 2,5 - Dimethylcyclohexan - 2,5 - dihydroperoxid, Cumolhydroperoxid, 2,5 - Dimethylcyclohexanon - 2,5 - dihydroperoxid, Diisopropylbenzolhydroperoxid, tert. - Butylhydroperoxid, p - Menthanhydroperoxid und 4 - Methylphenylisopropylhydroperoxid besteht.

6. Testmittel nach Anspruch 5, dadurch gekennzeichnet, daß das Hydroperoxid aus 2,5 - Dimethylcyclohexanon - 2,5 - dihydroperoxid oder Cumolhydroperoxid besteht.

7. Testmittel nach einem der Ansprüche 1 bis 6 in Form einer Lösung, die die Komponenten (A), (B) und (C) enthält.

8. Testmittel in Form eines Testpapiers, das durch Imprägnieren eines Substrats mit der Lösung nach einem der Ansprüche 1 bis 7 erhalten wurde.

**Revendications**

1. Agent pour test de détection du glucose ou du sang masqué dans un échantillon d'un fluide corporel qui peut également contenir une substance réductrice, qui comprend

(A) (i) un hydroperoxyde ou du peroxyde d'hydrogène (pour le test du sang masqué) ou

(ii) une peroxydase et une glucose-oxydase (pour le test du glucose)

(B) une quantité efficace, suffisante pour oxyder la substance réductrice dans ledit fluide corporel, de $NaIO_4$ et

(C) un colorant qui sert à détecter l'oxygène produit par la réaction de la peroxydase ou d'une substance du type peroxydase.

2. Agent pour test selon la revendication 1, dans lequel ladite quantité efficace de $NaIO_4$ est comprise dans l'intervalle allant de 0,3 à 1 000 m · moles/litre.

3. Agent pour test selon la revendication 1, dans lequel ledit agent pour test est une solution d'un agent contenant ledit colorant et $NaIO_4$; ledit $NaIO_4$ est présent en une quantité comprise entre 0,3 et 1 000 m · moles/litre; et ledit colorant est au moins un représentant choisi dans le groupe comprenant les composés de type benzidine, les azines hétérocycliques, les phénols et les constituants de résine de gaïac.

4. Agent pour test selon la revendication 3, dans lequel ledit $NaIO_4$ est présent en une quantité comprise entre 0,5 et 40 m · moles/litre et dans lequel ledit colorant est l'o-tolidine ou le phénol.

5. Agent pour test selon l'une quelconque des revendications 1 à 4, dans lequel ledit constituant (A) est un hydroperoxyde choisi dans le groupe comprenant le 2,5 - diméthylcyclohexane - 2,5 - dihydroperoxyde, l'hydroperoxyde de cumène, le 2,5 - diméthylcyclohexanone - 2,5 - dihydroperoxyde, l'hydroperoxyde de diisopropylbenzène, l'hydroperoxyde de t-butyle, l'hydroperoxyde de p-menthane et l'hydroperoxyde de 4 - méthylphénylisopropyle.

6. Agent pour test selon la revendication 5, dans lequel ledit hydroperoxyde est le 2,5 - diméthylcyclohexanone - 2,5 - dihydroperoxyde ou l'hydroperoxyde de cumène.

7. Agent pour test selon l'une quelconque des revendications 1 à 6, sous la forme d'une solution qui comprend les constituants (A), (B) et (C).

8. Agent pour test qui se présente sous la forme d'un papier réactif produit par imprégnation d'un substrat avec la solution selon l'une quelconque des revendications 1 à 7.

# FIG. 1

# FIG. 2

EP 0 141 244 B1

FIG.3

FIG.4

2